# EUROPEAN PATENT SPECIFICATION

(11) **EP 4 438 008 B1**
(45) Date of publication and mention of the grant of the patent: **04.11.2026**
(21) Application number: 24174470.5
(22) Date of filing: 08.11.2016
(51) Int. Cl.: A61F 2/24

(54) **IMPLANT DELIVERY CAPSULE**
IMPLANTATEINFÜHRUNGSKAPSEL
CAPSULE DE MISE EN PLACE D'IMPLANT

(30) Priority: 10.11.2015 US 201562253464 P; 04.11.2016 US 201615344163
(43) Date of publication of application: 02.10.2024
(62) Document number(s) of the earlier application(s) in accordance with Art. 76 EPC: 16864836.8 / 3 373 861
(73) Proprietor: Edwards Lifesciences Corporation, Irvine, CA 92614 (US)
(72) Inventor: Tamir, Ilan, IRVINE, CA, 92614 (US); Nir, Noam, IRVINE, CA, 92614 (US); Manash, Boaz, IRVINE, CA, 92614 (US); Maimon, David, IRVINE, CA, 92614 (US); Cohen, Oren, IRVINE, CA, 92614 (US)
(74) Representative: Eisenführ Speiser

(56) References cited:
- WO-A1-2015/127283
- US-A1- 2013 310 928

## Description

### FIELD

The present application concerns embodiments of a capsule for delivering a prosthetic device, and in particular capsules for delivery of prosthetic heart valves.

### BACKGROUND

Endovascular delivery catheter assemblies are used to implant prosthetic devices, such as prosthetic heart valves, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature. An introducer sheath generally has an elongated sleeve that is inserted into the patient's vasculature and a housing with one or more sealing valves that allows a delivery apparatus to be placed in fluid communication with the vasculature while preventing blood loss. A conventional introducer sheath typically requires a tubular loader to be inserted through the seals in the housing to provide an unobstructed path through the housing, thereby allowing a prosthetic device to be passed through the introducer sheath.

Certain approaches through the heart for mitral valve replacement can be particularly challenging for delivery of prosthetic mitral valves due to the sharp turns needed near the end of the introducer sheath. An example of a stent-mounted mitral valve prosthetic is described in U.S. Patent No. 8,449,599. FIG. 67 of the '599 patent (replicated herein as FIG. 1) illustrates delivery of a prosthetic mitral valve via a transseptal approach. More specifically, an incision is made in the atrial portion of a septum 30 for allowing access from a right atrium 26 to a left atrium 4. A sheath 2102 of a delivery catheter 2100 is inserted into the left atrium 4 and bent sharply in the direction of the mitral valve. Once in the left atrium 4, the distal end of the sheath 2102 is moved across the mitral annulus such that ventricular anchors 126 of the prosthetic valve 100 are positioned beyond the mitral leaflets 10, 12. The prosthetic valve is expelled from the distal end of the sheath 2102 using pusher shaft 2106.

Despite the improvements provided by the '599 patent, the sharp bend in the introducer sheath (prior to reaching the mitral valve) increases the difficulty of withdrawing the sheath relative to the pusher and mitral valve. The sheath has to be retracted backwards around two bends. Also, there is limited space in the heart chambers (left ventricle and left atrium) for relative movement of the introducer sheath and the deploying prosthetic mitral valve. The lack of space makes it difficult for the cardiologist to deliver the prosthetic mitral valve.

US 2013/310928 A1 discloses a percutaneously implantable prosthetic heart-valve system comprising an expandable support and a plurality of asymmetrically arranged, flexible arms that extend from the support to capture native leaflets and engage the sub-annular surface. Thus, there remains a need for improvements to delivery systems for prosthetic mitral valves, especially for transseptal approaches.

### SUMMARY

Disclosed herein are embodiments of delivery capsules used for delivering prosthetic implants around the relatively tight bends of a catheter. In particular, disclosed herein are delivery capsules including a tubular layer for holding the heart valve and a frame that is held in a curled configuration by an elongate element. Withdrawal of the elongate element releases the frame to expand into a larger diameter. Another embodiment includes a helical wire wrapped around the capsule for easy delivery through bends and a second helical wire that is advanced between windings of the first helical wire to selectively stiffen the capsule for withdrawal. Other embodiments include sock-like capsules that can be crumpled into shorter lengths by pulling a pull line attached to the capsule.

In the embodiments disclosed herein, a delivery system extends through the vasculature to access a native heart valve. The delivery systems include a catheter configured to extend through the vasculature. The catheter has a distal end for positioning in proximity to the native heart valve. The delivery systems also include an expandable prosthetic heart valve coupled to the distal end of the catheter for placement within the native heart valve.

Some embodiments of the delivery system disclosed herein include a tubular layer defining a lumen for containing the prosthetic heart valve, a frame coupled to the tubular layer and encircling the tubular layer, and a thin, elongate element extending through the frame. In some embodiments, the frame is comprised of a stiff fabric. The elongate element holds the frame in an inwardly curled configuration and against an outward bias of the frame. The inwardly curled configuration narrows the lumen and holds the tubular layer and prosthetic heart valve in a crimped configuration. The elongate element is further removable from the frame to release the tubular layer and frame into an expanded configuration. In some embodiments, the thin elongate element is a rod extending through a lumen defined by the catheter.

The frame can include a plurality of fingers, each of the fingers defining an opening. A first set of the fingers can extend in a clockwise direction and a second set of the fingers can extend in a counter-clockwise direction. The elongate element extends through the opening of each of the fingers to hold the frame in the inwardly curled configuration. The first set of fingers and second set of fingers can be engaged to axially align the openings with the elongate element passing therethrough in the inwardly curled configuration, and then disengaged with sliding removal of the elongate element. This disengagement causes the frame to assume the expanded configuration.

The frame can also include a base sheet. The first set of fingers extends from a first longitudinal edge of the base sheet and the second set of fingers extends from a second longitudinal edge of the base sheet opposite the first longitudinal edge. In some implementations, each of the fingers has a free end defining the opening, and the free end may include a loop defining the opening.

In some implementations, the frame and tubular layer are configured to remain with the expandable prosthetic valve upon removal of the catheter. The prosthetic valve can include a plurality of tissue leaflets secured to the tubular layer and frame.

In some implementations, the frame includes a helical wire helically extending along the tubular layer. The helical wire may define a loop at one end. The elongate element engages the loop and applies tension to the helical wire to hold the helical wire in the inwardly curled configuration. The elongate element may be, for example, a suture extending through a lumen of the catheter.

Other embodiments of the delivery system disclosed herein include a flexible capsule defining a lumen. The capsule is configured to contain the prosthetic heart valve. It includes a tubular layer and a capsule helical wire attached to and encircling the tubular layer. In some implementations, the tubular layer includes a thin polymer encapsulating the capsule helical wire. An inside surface of the tubular layer can, in some examples, be smooth. The capsule helical wire has a helical pitch space defined between adjacent windings of the capsule helical wire. In some implementations, the helical pitch space is defined on an outside surface of the tubular layer.

The delivery systems of this embodiment also include a torque shaft having a proximal end and a distal end and a proximal helical wire coupled to the distal end of the torque shaft. The proximal helical wire has a diameter and pitch matching the helical pitch space. Rotation of the proximal end of the torque shaft rotates the proximal helical wire and axially advances the proximal helical wire into the pitch space of the capsule helical wire so as to stiffen the capsule for retrieval of the prosthetic heart valve into the lumen.

The delivery systems of this embodiment can also include a delivery sheath having a lumen containing the proximal end of the catheter. The capsule helical wire can be configured to reduce in diameter for withdrawal of the flexible capsule into the lumen of the delivery sheath. An elongate puller can be coupled to a distal end of the flexible capsule and configured to pull the proximal end of the flexible capsule to reduce the helical pitch space and reveal the prosthetic heart valve.

Other embodiments of the delivery system disclosed herein can include a delivery sheath. The delivery sheath is configured to extend through the vasculature, and has a proximal end and a distal end (the distal end for positioning in proximity to the native heart valve). The delivery sheath defines a lumen, and the catheter extends through the lumen of the delivery sheath. Also included is a tubular capsule for containing the expandable prosthetic heart valve. The tubular capsule includes a flexible wall material (such as a woven material) defining a lumen, a proximal end, and a distal end. A pull line extending through the lumen of the delivery sheath is attached to one of the ends (proximal or distal) of the tubular capsule, and configured to pull on the tubular capsule. The flexible wall material is configured to crumple in response to pulling by the pull line to reveal the expandable prosthetic heart valve. The delivery systems of this embodiment may further include a nose positioned distal to the tubular capsule. The pull line can extend through the nose and doubles back to attach to the proximal end of the tubular capsule to pull the proximal end of the tubular capsule distally.

Other embodiments of the delivery system disclosed herein can include an expandable prosthetic valve having a cylindrical frame and at least one paddle, and a tubular capsule with a proximal end and a distal end. The tubular capsule includes a flexible wall material having an inner layer extending around the cylindrical frame and an outer layer extending around the paddle. A pulling element is attached to the proximal end of the tubular capsule and extends proximally along the catheter. Pulling proximally on the tubular capsule using the pulling element first releases the paddle and then releases the cylindrical frame.

For the delivery systems of this embodiment, the inner layer can have a length greater than the outer layer. The inner layer can also have a crumple zone configured to be taken up without movement of a distal end of the inner layer while the outer layer releases the paddle. The distal end of the inner layer may moves once the crumple zone is taken up to release the cylindrical frame. In some implementations, the inner layer has a free end and the outer layer has a free end. In some implementations, the tubular capsule extends distally to a distal bend and extends proximally from the distal bend to define the outer layer to a proximal bend then extends distally from the proximal bend under the paddle and over the cylindrical frame to define the inner layer.

### DESCRIPTION OF DRAWINGS

FIG. 1 is a sectional view of a heart with a delivery system delivering a prosthetic valve into a native mitral valve;
FIG. 2 is a plan view of a distal end of a mitral valve delivery system;
FIG. 3 is a schematic of a capsule of one embodiment of the present disclosure including a helical thread wound around a prosthetic valve frame;
FIG. 4 is a schematic of a capsule of another embodiment of the present disclosure including fingers holding a prosthetic valve in a crimped configuration;
FIG. 5 is a schematic of the capsule and prosthetic valve of FIG. 4 in an expanded configuration;
FIG. 6 is a perspective view of a capsule frame of another embodiment of the present disclosure including interdigitating fingers curled into a crimped configuration;
FIG. 7 is a plan view of the capsule frame shown in FIG. 6;
FIG. 8 is a perspective view of the capsule frame of FIG. 6 attached to a prosthetic valve frame body and in an expanded configuration;
FIG. 9 is a side elevation view of another embodiment of a capsule including a helical spring frame;
FIG. 10 is a side elevation view of the capsule of FIG. 9 in an axially compressed condition;
FIG. 11 is a partial sectional view of a distal end of a delivery catheter retrieving the proximal end of the capsule of FIG. 9;
FIG. 12 is a side elevation view of the capsule of FIG. 9 abutting a proximal spring;
FIG. 13 is a side elevation view of the capsule and proximal spring of FIG. 12, wherein the proximal spring has been rotationally advanced into the pitch spaces of the capsule;
FIG. 14 is a side elevation view of a frame of a prosthetic mitral valve in a deployed configuration;
FIG. 15 is a side elevation view of the frame of FIG. 14 in a crimped configuration with paddles spaced away from a body of the frame;
FIG. 16 is a partial sectional view of a sock-like capsule embodiment , according to the invention, with multiple layers holding a prosthetic mitral valve in a crimped configuration;
FIG. 17 is a partial sectional view of the capsule of FIG. 16 with a first outer layer withdrawn to allow actuation of the paddles of the prosthetic mitral valve;
FIG. 18 is a partial sectional view of a second inner layer of the capsule of FIG. 16 withdrawn and the prosthetic mitral valve in an expanded configuration;
FIG. 19 is a partial sectional view of a multi-layer capsule of another embodiment of the present invention;
FIG. 20 is a partial sectional view of a sock-like capsule of another embodiment of the present invention having bends for holding a mitral valve with paddles in a crimped configuration;
FIGS. 21 and 22 are partial sectional views of the capsule in FIG. 20 partially withdrawn to remove the bends and free the paddles for expansion;
FIG. 23 is a partial sectional view of another embodiment of a capsule having bends;
FIG. 24 is a side elevation view of another embodiment of a capsule of the present disclosure having a woven material in a sock-like configuration;
FIG. 25 is a side elevation view of the capsule of FIG. 24 being folded or bunched as it is withdrawn proximally by pulling elements;
FIG. 26 is a side elevation view of another sock-like capsule embodiment having pulling elements extend through a nose cone for folding the capsule in the distal direction;
FIG. 27 is a side elevation view of the capsule of FIG. 26 progressively folding in the distal direction to release a prosthetic valve for expansion; and
FIGS. 28-29 are schematics of a standalone, sock-like capsule of another embodiment of the present disclosure.
FIGS. 30-31 are perspective views of capsule frames of another embodiment of the present disclosure including multiple suture lines for staged deployment.

### DETAILED DESCRIPTION

The invention is defined in the appended claims. The following description of certain examples of the inventive concepts should not be used to limit the scope of the claims. Other examples, features, aspects, embodiments, and advantages will become apparent to those skilled in the art from the following description. Accordingly, the drawings and descriptions should be regarded as illustrative in nature and not restrictive.

For purposes of this description, certain aspects, advantages, and novel features of the embodiments of this disclosure are described herein. The described methods, systems, and apparatus should not be construed as limiting in any way. Instead, the present disclosure is directed toward all novel and nonobvious features and aspects of the various disclosed embodiments, alone and in various combinations and subcombinations with one another. The disclosed methods, systems, and apparatus are not limited to any specific aspect, feature, or combination thereof, nor do the disclosed methods, systems, and apparatus require that any one or more specific advantages be present or problems be solved.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

Disclosed herein are embodiments of delivery capsules for delivering prosthetic implants around the relatively tight bends of a catheter. In particular, disclosed herein are delivery capsules including a tubular layer for holding the heart valve and a frame that is held in a curled configuration by an elongate element, such as a suture, a rod, or a wire. Withdrawal of the elongate element releases the frame to expand into a larger diameter. Another embodiment includes a helical wire wrapped around the capsule for easy delivery through bends and a second helical wire that is advanced between windings of the first helical wire to selectively stiffen the capsule for withdrawal. Other embodiments include sock-like capsules that can be crumpled into shorter lengths by pulling a pull line attached to the capsule.

The capsules disclosed herein may also be used to deliver various types of implantable devices, such as self-expanding implantable heart valves, stents or filters. The terms "implant" and "implantable" as used herein are broadly defined to mean anything - prosthetic or not - that is delivered to a site within a body. A diagnostic device, for example, may be an implantable. The term "implant" as used herein also does not need to be a permanent implant - for example a balloon is an implant temporarily - but could be any device delivered into the body for a procedure. The disclosed capsules are, however, well adapted for use with self-expanding prosthetic heart valves and in particular prosthetic heart valves delivered into particularly tight spaces or through tortuous pathways.

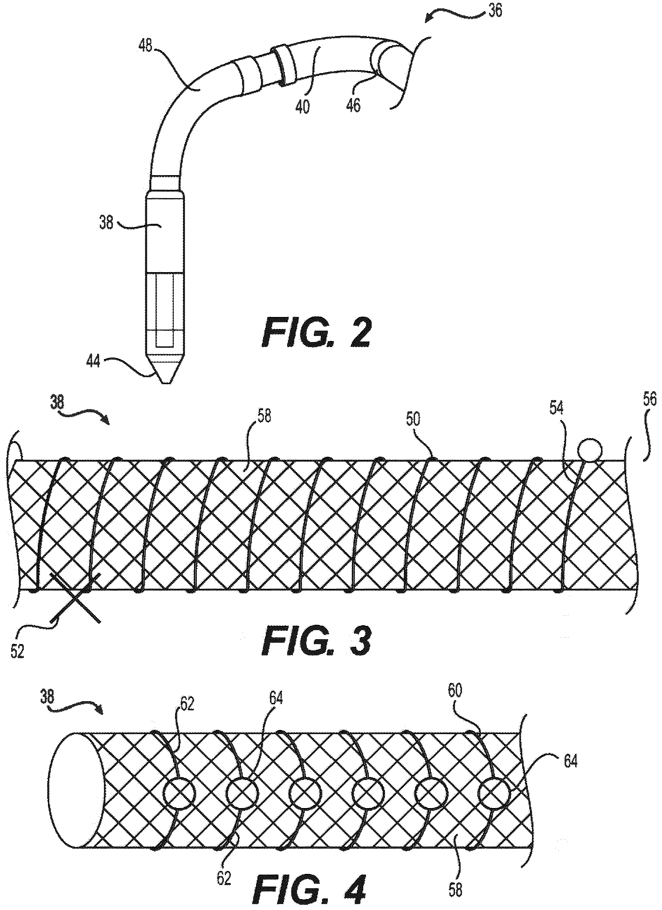

FIG. 2 shows a distal end of a delivery catheter 36 generally having a tubular structure and including a capsule 38 and a nosecone 44. The delivery catheter 36 includes a first bend 46 and a second bend 48 before reaching the distally positioned capsule 38. The first bend 46 can be due to a steering component bending a normally straight portion of the tubular structure or pre-bent, biased portion of the tubular structure assuming its natural shape. The second bend 48 similarly may be bent by steering, or assuming a pre-bent shape. For example, the portion defining the second bend 48 may be extended out of the more proximal portion of the tubular structure defining the first bend 46 and then, under bias of a pre-bent wall structure, may assume the bent shape. Regardless, the bends 46, 48 illustrate one of many configurations in which embodiments of the present disclosure are advantageous - tight or confined spaces where delivery/retrieval occurs proximate a bend in the catheter or other delivery device.

The capsule 38 shown in FIG. 2 generally represents any of the capsule embodiments disclosed herein. The capsule is configured to mate with the nosecone 44 to define a receptacle for delivery of an implant, such as a self-expanding prosthetic heart valve in a crimped configuration. The capsule 38 can be delivered by pushing an inner catheter tubular structure - such as the one defining the second bend 48 - out of a delivery sheath or other tubular structure - such as the one defining the first bend 46 in FIG. 2. Once the capsule 38 is free, generally, the capsule needs to be somehow retracted, removed or disengaged from its position covering and/or restraining the implant. Various embodiments disclosed herein below facilitate retraction or removal or disengagement without requiring a large space and/or with a proximate sharp bend in the tubular structure of the delivery catheter 36.

FIG. 3 shows one embodiment of a capsule 38 formed of a helical suture or wire frame 50 wrapped around a sheath or, in the illustrated case, a valve frame 58 containing a tissue valve (e.g., made from pericardium). The helical wire frame 50 is comprised of a wire that is fixed at a distal end 52 to a distal portion of the valve frame 58 and wraps around the outside of the valve frame. The helical wire frame 50 also includes a proximal end 54 having defined thereon a loop or other feature that facilitates attachment of a suture 56 or other elongate member. Deployment is achieved by releasing tension in the helical wire frame 50 and detaching the suture 56 from the wire frame. The release of tension allows the helical wire frame 50 to expand to an enlarged, biased shape. Or, the release of tension in the helical wire frame 50 - if the wire frame is unbiased, such as by being comprised of suture - can allow a self-expanding valve frame 58 to expand into position within a native mitral valve annulus.

In a sense, the capsule 38 is partially formed by the valve frame 58, or the capsule could be regarded as being the helical wire frame 50 standing alone. The capsule could also be attached to a dedicated tubular cover that is part of the capsule 38. Of course, an advantage of having the capsule comprise the helical wire frame 50 standing alone is that the helical wire frame 50 can be left in place in the implant location. It does not have to be withdrawn into the second bend 48 of the delivery catheter 36. Only the suture 56 need be withdrawn and it easily pulls back into the delivery catheter 36, even around tight bends.

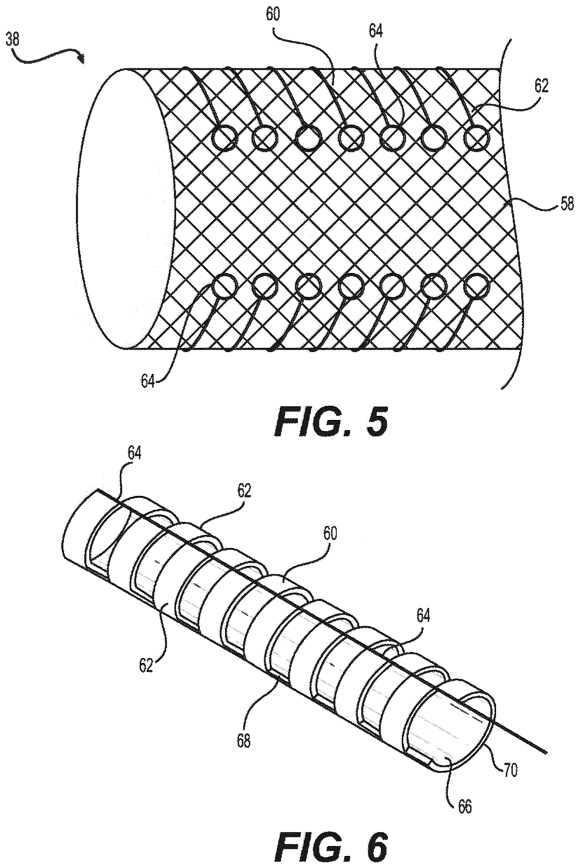

FIGS. 4 and 5 show another embodiment, wherein the capsule 38 includes a differently configured wire capsule frame 60 including a plurality of fingers 62. The fingers 62 extend circumferentially around the valve frame 58 and have ends including loops 64. The fingers 62 thus have a C-shape with free ends having the loops 64. The fingers 62, in the embodiment of FIG. 4, are axially spaced along the outside of the valve frame 58. The loops 64 at the ends of the fingers are in overlapping, paired relationships on the outside surface of the valve frame 58. Also, the pairs of loops 64 are positioned in alignment extending in the axial direction of the valve frame 58. A suture or wire (not shown) can extend through the aligned pairs of loops to hold the fingers 62 in a smaller diameter or inwardly curled configuration in FIG. 4. Holding the fingers in the smaller diameter configuration also holds the valve frame 58 in a crimped condition for delivery.

FIG. 5 shows an expanded configuration of the valve frame 58, wherein the suture 56 has been removed from engagement of the overlapping pairs of loops 64. As each loop pair is disengaged, the bias of the fingers 62 and/or the valve frame 58 urges the valve frame into the expanded configuration. Like the embodiment of FIG. 3, withdrawal of just the suture 56 or other elongate element is relatively easy and the fingers 62 remain with - and contribute to - the valve frame 58.

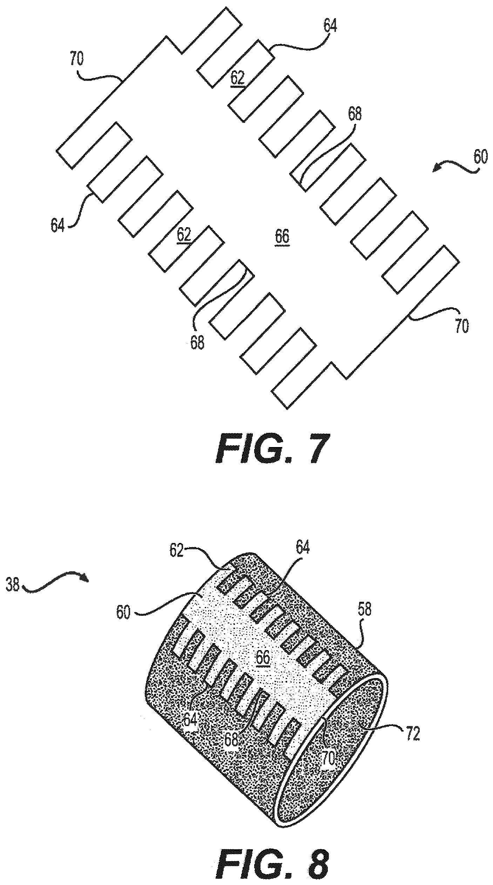

FIGS. 6 through 8 illustrate yet another embodiment, wherein the frame 60 includes fingers 62 that extend in clockwise and counterclockwise directions (when viewed along the axis of the valve frame 58). The frame 60 also includes a base sheet 66 having a rectangular shape with a pair of longitudinal edges 68 and circumferential edges 70. The circumferential edges 70 extend between the longitudinal edges, as shown in FIG. 7. The fingers 62 extend from the longitudinal edges 68 and are spaced there along - with the fingers on one of the edges out of phase in their spacing with the fingers on the other one of the edges. Thus, when the base sheet 66 is rolled up into a tubular configuration, as shown in FIG. 6, the oppositely facing fingers extend between each other or "interdigitate." The dimensions of the fingers 62 relative to the base sheet may vary. For example, the capsule may have a narrow base sheet 66 and long fingers 62, or vice versa having a wide base sheet and short fingers.

Each of the fingers 62 includes a loop 64 at its free edge. Therefore, when the fingers are interdigitated, the loops are aligned on the outside surface of the valve frame 58. The suture 56, or other elongate member - such as a wire or rod - extends through the aligned loops to hold the frame 60 in the curled configuration shown in FIG. 6. FIG. 8 shows the valve frame 58 in the expanded configuration after withdrawal of the rod 56 from the aligned loops 64. After withdrawal of the rod, the base sheet 66 and fingers 62 expand toward their flat configuration, as shown in FIG. 7. Even in the expanded condition, the base sheet 66 and fingers 62 are not entirely flat and so continue to exert a bias urging the valve frame 58 to its largest diameter. This helps to seat and or maintain positioning of the valve frame 58 in the native mitral valve annulus, for example.

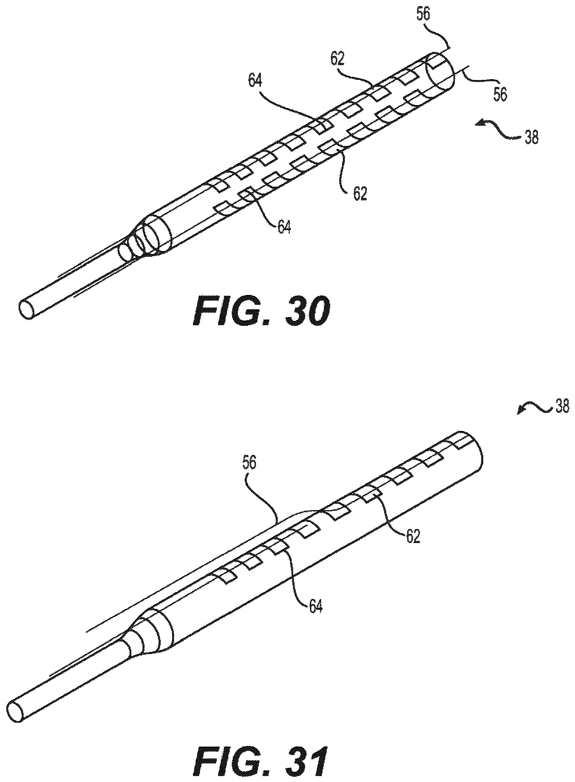

FIGS. 30 and 31 show embodiments of capsule 38 that include multiple frames 60 with fingers 62 terminating in loops 64 at their free edges. Two or more arrays positioned radially around the circumference of the capsule may be deployed separately by distinct sutures 56, allowing for a directionally defined and staged deployment. In FIG. 31, distinct sutures 56 are attached along separate lengths of the array of fingers 62 and loops 64. Staged release of the distinct sutures 56 enables staged control of deployment along the length of the capsule.

The frames 50, 60 (and other features described herein benefiting from elastic memory) can be constructed of materials with some stiffness and elasticity. For example, the frames could be constructed of a super elastic nitinol which is capable of higher elastic strains that can be present in the curled configurations (when the valve is in a crimped condition). At the same time, the elasticity of the nitinol allows it to expand back into its memory shape, such as the flat configuration or the larger diameter C-shapes shown in the figures. Other materials could also be used such as more pliable polymers, depending, for example, upon the desired amount of elastic bias contributed by the capsule frames versus the valve frame 58. Polymers may include nylon, polyethylene terephthalate (PET), expanded polytetrafluoroethylene (ePTFE), or ultrahigh-molecular-weight-polyethylene based materials such as, for example, DYNEEMA fibers.

The valve frames can include circumferential fabric tubes - such as the tubes shown in FIGS. 5 and 8. The circumferential fabric tubes can be configured to block blood flow around the valves or other valve frame components (such as stents). The fabric tubes also do not substantially resist expansion of the capsule or valve frames once released by withdrawal of the suture 56. Advantageously, the capsule 38 can remain as part of the implanted valve and does not require withdrawal back through the delivery system. As a result, deployment can be performed with lower forces - retracting a capsule from over the valve frame (as in conventional systems) generates friction forces. The capsule can be a much larger diameter because it need not be withdrawn back through the delivery system.

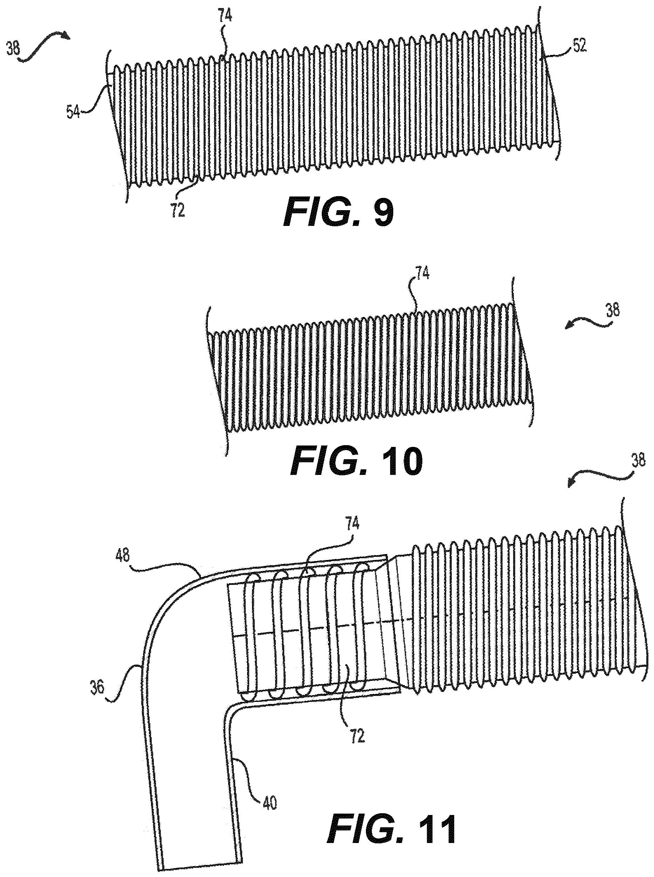

FIGS. 9 through 13 illustrate an embodiment of capsule 38 including a tubular layer 72 and a capsule helical wire 74. The tubular layer 72 defines a lumen within which the self-expanding prosthetic heart valve can be restrained for delivery. The tubular layer can be constructed of materials, such as a PTFE material, elastomer, a semi-compliant polymer or woven materials, which are flexible and relatively impermeable to fluids.

The capsule helical wire 74, as shown in FIG. 9, includes a flexible helical wire that is attached to and encircles the tubular layer 72. The capsule helical wire 74 has a helical pitch space defined between adjacent windings of the helical shape as it extends along the outside surface of the tubular layer. The capsule 38 with the helical wire can advantageously compress, as shown in FIG. 10, to reduce the helical pitch space to zero. This shortens and stiffens the capsule 38 for advancement against friction within the delivery sheath and/or body lumens.

Also, the helical structure of the helical wire 74 facilitates easy withdrawal, as shown in FIG. 11, into the second bend 48 of the delivery catheter 36. The helical structure can lengthen, resulting in reduction of cross-section of the capsule 38. The pitch of the capsule helical wire 74 can vary depending on the desired shortening of the capsule 38.

The tubular layer 72 can be sufficiently thin and compliant to provide for a smooth inside diameter but a protruding shape of the wire 74 on the outside diameter. This creates a ribbed appearance for the outside of the capsule 38. This configuration could also be reversed, smooth on the outside and ribbed on the inside.

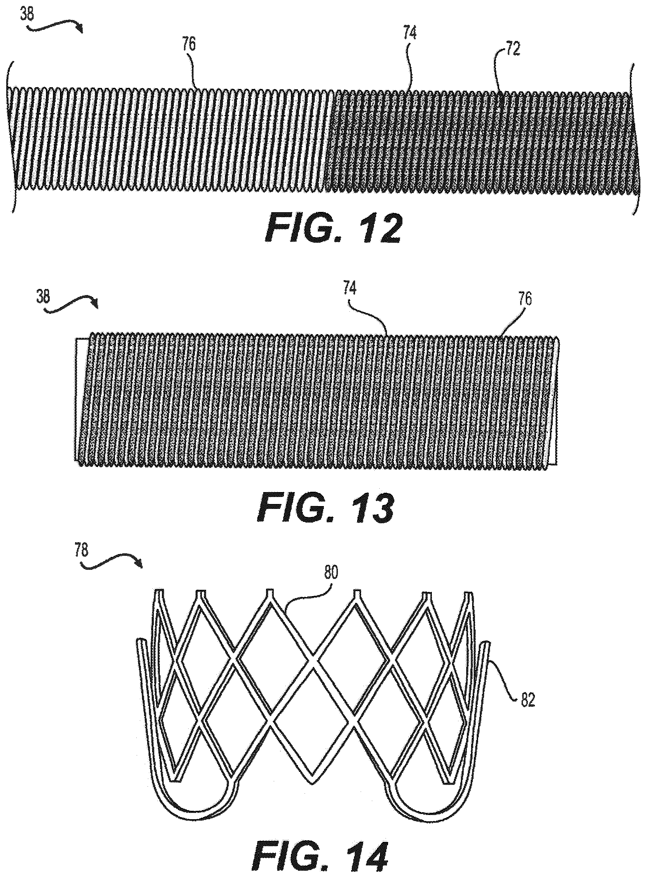

The delivery catheter 36 can also include a torque shaft and a proximal spring or helical wire 76 with a similar or slightly larger diameter than the diameter of the capsule 38, as shown in FIG. 12. The proximal helical wire 76 can be connected to the distal end of the torque shaft. The torque shaft (not shown) extends through the tubular structure of the delivery catheter 36 and is configured to impart torque applied at its proximal end onto distal end attachments, such as the proximal helical wire 76. The proximal helical wire 76 and torque shaft are separate from the capsule 38 to facilitate relative movement between the two. The proximal wire 76 may also be mounted on the delivery system tubular structure, behind the capsule 38, in a way that it is restricted from being torqued or moved proximally, but is still free to move distally.

The proximal helical wire 76 has a wire diameter and pitch matching the helical pitch space of the capsule helical wire 74. The distal end of the proximal helical wire 76 is positioned at the proximal end of the capsule helical wire 74. Thus, rotation of the torque shaft rotates the proximal helical wire 76 and advances it into the helical pitch space, as shown in FIG. 13. Advancement of the proximal helical wire 76 into the helical pitch space of the capsule helical wire 74 progressively stiffens the capsule. As another option, the shaft attached to the capsule 38 can be rotated while pulling the capsule backwards against the proximal helical wire 76. This causes the proximal helical wire 76 to climb over the capsule 38 and the capsule can be removed through the delivery system catheter 36.

The proximal helical wire 76 can also have a tubular layer with an encapsulation similar to the capsule helical wire 74, but in a reverse configuration - the inside diameter ribbed and outside diameter smooth. In this manner, the ribbed profiles can face each other for advancement of the two coils within their respective pitches. The proximal helical wire 76 can be round or flat or other shaped cross-section wire, but with the same diameter as the pitch spaces of the capsule helical wire 74. The term "wire" as used herein is also not limited to wire of a drawn configuration - instead it could be wire cut or etched from a tube for example.

Generally, the capsule 38 of FIGS. 9 through 13 provides the ability to change the structural properties of the capsule while in use and per the needs of the procedure. Advantages of the use of the capsule helical wire 74 include the flexibility to track through the tortuous path (bends) of the delivery catheter 36 on the way to the mitral valve. The flexibility also helps in the limited room in the left atrium, where it is normally difficult to pull the capsule back in a straight line to expose the prosthetic mitral valve. Also, the capsule 38 may have an outer diameter that is the same or similar to the outer diameter of the tubular wall structure of the delivery catheter 36 and still be able to be withdrawn - thus allowing for less crimped or larger implants. Also, the capsule helical wire 74 can be compressed through a bellows effect when pulled proximally from its distal side, as shown in FIG. 10, allowing partial or full valve deployment in the tight confines of the left atrium.

After the valve is deployed, the capsule 38 is empty for pulling proximally into the catheter 36 even if it has a larger diameter. The coil configuration with the semi-compliant tubular layer 72 facilitates reduction of the capsule diameter by passively increasing the pitch distance between the coils, as shown in FIG. 11. The larger diameter capsule 38 allows delivery of larger profile implants. Further, being able to selectively stiffen the capsule, through advancement of the proximal helical wire 76, enables a "bail out" procedure where the capsule 36 is advanced back over the implant in an attempt to retrieve it.

The wires used in the helical constructions can be polymer, metal or other materials. For example, super elastic nitinol wire works particularly well given its large range of elastic strain deformation.

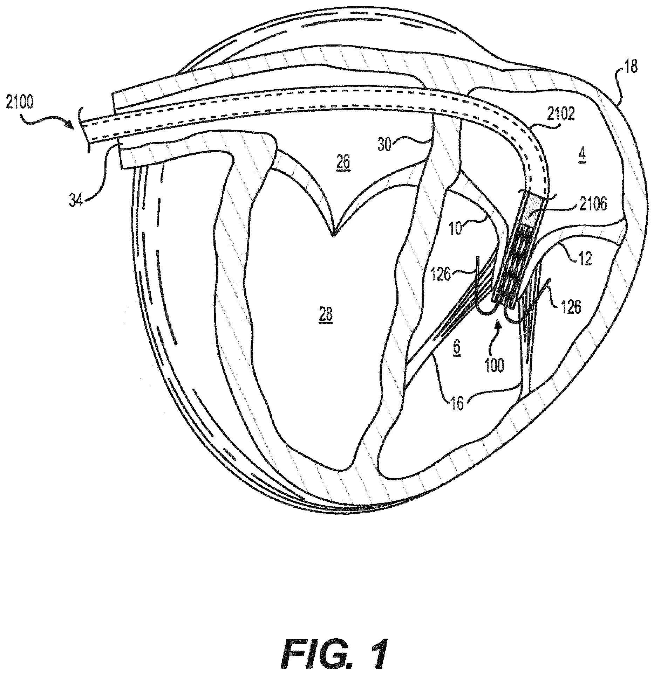

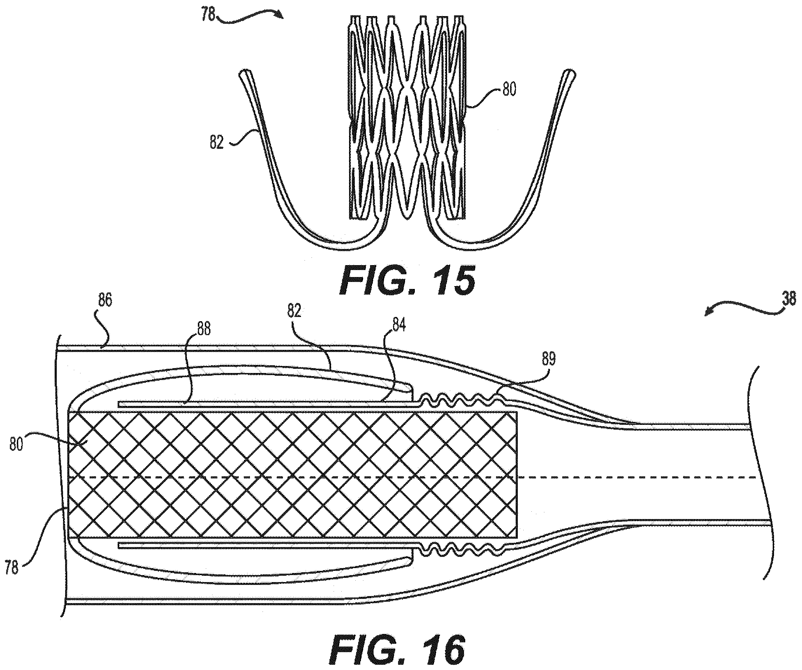

FIGS. 14 and 15 show a prosthetic mitral valve frame 78 that includes a cylindrical stent body 80 and a pair of paddles 82. Generally, the paddles 82 are configured to engage or anchor into the chordae tendineae 16, as shown in FIG. 1. FIG. 14 shows the stent body 80 in the expanded configuration, wherein the paddles 82 are folded up against the outside surface of the body. FIG. 15 shows the crimped stent body 80 with the paddles 82 extending outward and spaced from an outside surface of the body.

As shown in FIGS. 16 through 18, the delivery system can include capsule 38 with multiple woven layers configured to allow sequential deployment of the paddles 82 and then the stent body 80. The multiple layers include an inner layer 84 extending around the stent body 80 and an outer layer 86 extending around the paddles 82. In the delivery configuration of FIG. 16, the inner layer 84 includes a distal portion 88 that is straight (in cross section) and cylindrical, extending directly under the paddles 82. The paddles have free ends that abut the outer surface of the inner layer 84. The inner layer also includes a proximal portion 89 that defines a crumple zone. The crumple zone includes accordion folds or other types of bunching defining a loose accumulation of the inner layer 84.

The outer layer 86 extends over the outside of the paddles 82 and, in the delivery configuration, has a free end that extends further than the free end of the inner layer 80. At a proximal end, the inner and outer layers 84, 86 are bonded, formed or otherwise coupled together to become a single layer which can be pulled to remove the multi-layered capsule 38.

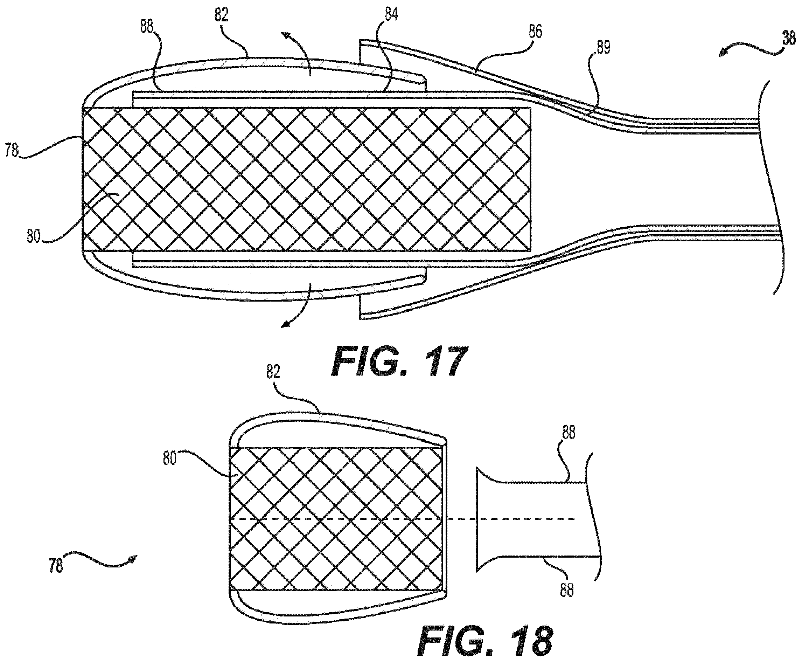

FIG. 17, for example, shows a first stage of withdrawal of the capsule 38 into the distal end of the delivery catheter 36. In particular, the outer layer 86 withdraws in proportion to the length of pull on the proximal end of the capsule 38 while the distal free edge of the inner layer 84 remains static as the crumple zone of the proximal portion 89 straightens out. Movement of the outer layer 86 releases the paddles to open and engage the chordae tendineae. Then, as shown in FIG. 18, the proximal pulling of the capsule 38 finally pulls the free end of the inner layer 84 from the outside of the stent body 80 so that it can expand into place, such as within the native mitral valve annulus.

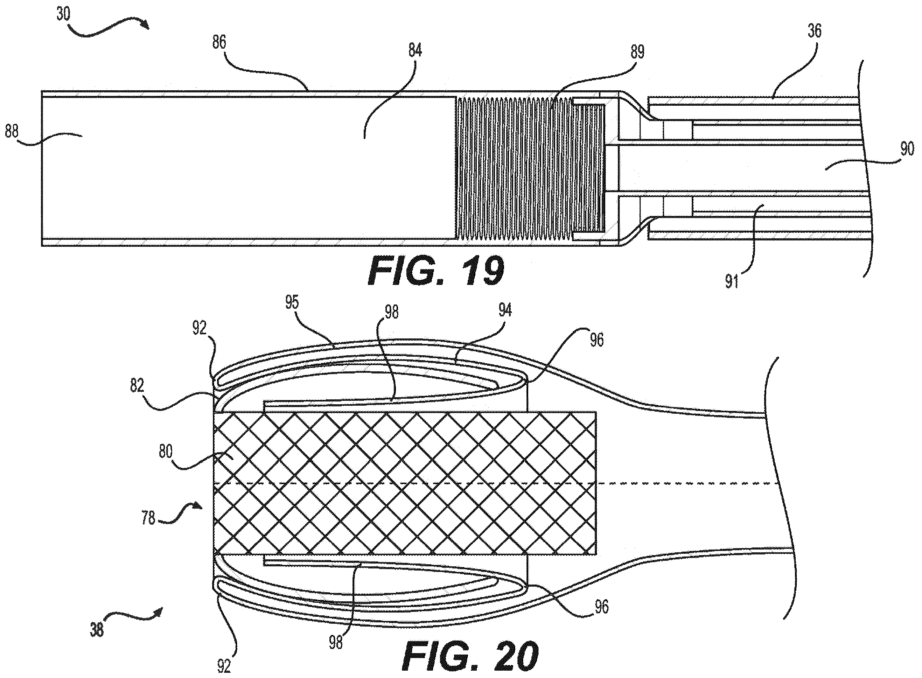

FIG. 19 shows a cross-section of capsule 38 with the crumple zone and the inner layer 84 extending around an inner shaft 90 for holding the prosthetic mitral valve (not shown). Also, the inner layer 84 and the outer layer 86 are attached to an outer shaft 91 that can be withdrawn independently of the inner shaft 90.

FIGS. 20 through 23 illustrate an embodiment of capsule 38 that uses bends in a long capsule to achieve a similar staged deployment. As shown in FIG. 20, the capsule 38 extends distally to a distal bend 92 and extends proximally from the distal bend to define an outer layer 94 (which is within an outermost layer 95). The capsule 38 continues to extend to a proximal bend 96. Then, the capsule 38 extends distally from the proximal bend 96, under the paddles 82 and over the stent body 80 to define an inner layer 98. The outer layer 94 thus restrains the paddles 82 in position and the inner layer 98 restrains the stent body 80 in its crimped position.

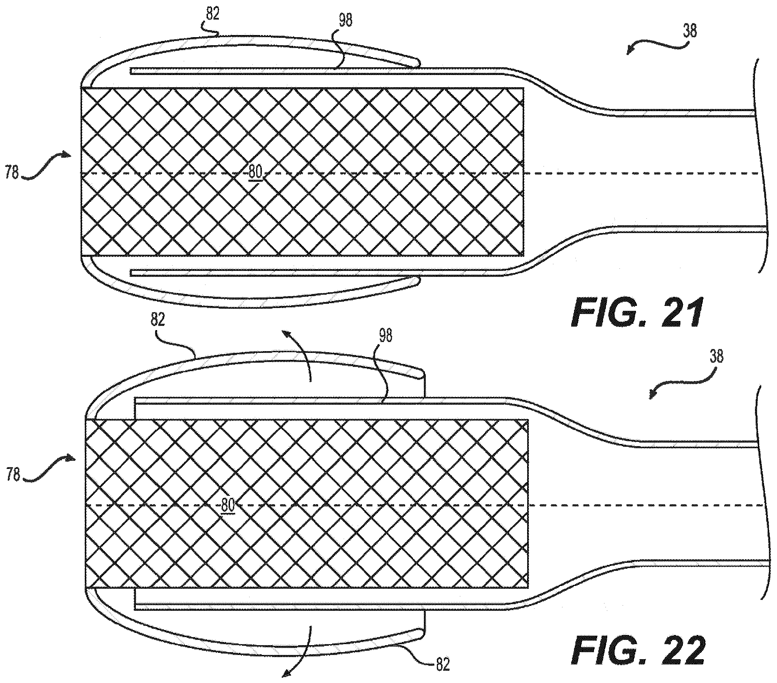

As shown in FIGS. 21 and 22, pulling proximally on the capsule 38 undoes the distal bend 92, and then proximal bend 96, to remove the outer layer 94 and release the paddles 82. Further pulling then retracts the remaining inner layer 98 from the stent body 80 so that it can expand into place within the mitral valve annulus.

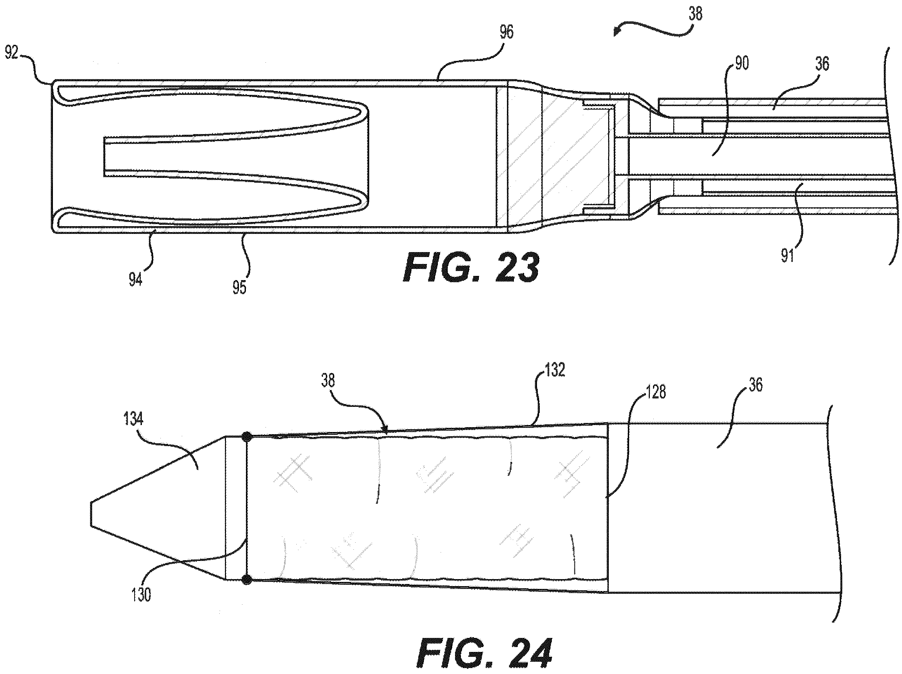

FIG. 23 shows the capsule with bends 92, 96 extending around the inner shaft 90 for holding the prosthetic valve and connected to the outer shaft 91. The outer shaft 91 can be withdrawn into the delivery catheter 36 independently of the inner shaft 90 for deployment of the prosthetic valve.

Advantageously, the capsule 38 with crumple zones or bends can be retrieved back into the delivery catheter 36 without changing the delivery system position and orientation within the heart. Also, the capsule 38 can be much larger in diameter than the delivery catheter 36 because it is constructed of a flexible material, such as a fabric, and can be retrieved through smaller openings and through catheter bends. Thus, the delivery system can employ smaller profile tubular wall structures and sharper bends. The capsule material can be very thin - 0.5 mm or less for example - due to the multiple layers employed. And the two-stage deployment of the prosthetic heart valve allows for more control of deployment.

Variations of the multi-layer capsule 38 include variations in diameter along the length to adapt to different valve shapes. The capsule 38 may be constructed of PET, nylon, DYNEEMA, metal wires or other flexible materials, alone and in combination.

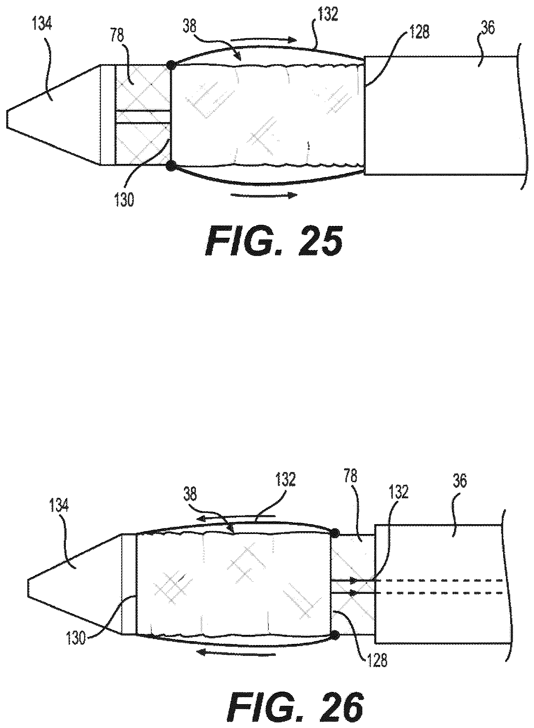

FIGS. 24-27 show sock-like embodiments of the capsule 38 having a flexible wall material with a proximal end 128 and a distal end 130. The capsule 38 can have pull wires 132 attached to one of the ends 128, 130 so as to crumple the flexible wall material into an axially compressed state. FIGS. 24 and 25 show the pull wires 132 extending out of a distal end of the catheter 36, over the outside of capsule 38 and attaching at the distal end 130 of the capsule. The pull wires, when pulled, crumple up the capsule 38 as the distal end 130 retracts back toward the proximal end 128 to reveal the underlying prosthetic mitral valve with minimal friction forces. The sock-like crumpling avoids the capsule friction against the inside of the delivery catheter and along the outside of the prosthetic mitral valve.

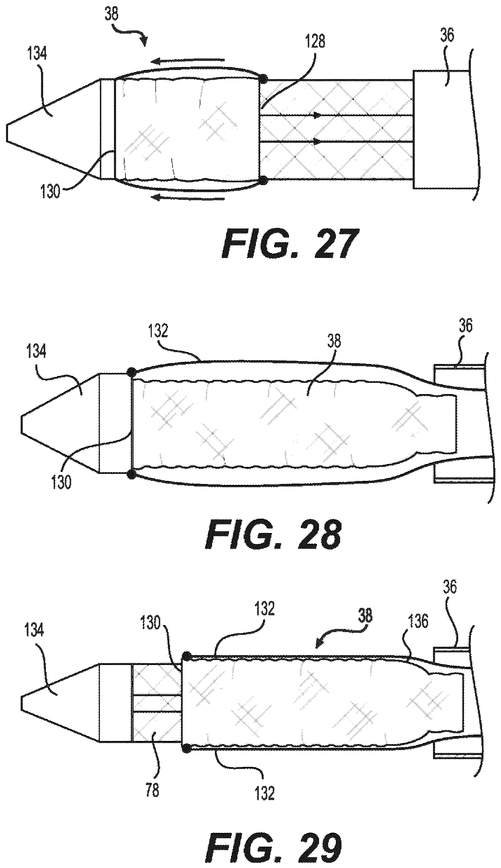

The pull wires 132 may also interact with a nosecone 134. FIGS. 26 and 27 show an embodiment wherein the pull wires are connected to the proximal end 128, extend distally to wrap around a feature (e.g., a pulley or post) inside a nosecone 134 to double-back through a center of the prosthetic mitral valve to the proximal end of the delivery catheter 36. Pulling on the pull wires 132 moves the proximal end 128 toward the distal end 130, crumpling up the sock-like capsule 38. This configuration advantageously allows the proximal end of the prosthetic valve to expand first, which can be advantageous for deployments in the direction of blood flow. The proximal end of the valve can expand as an anchor.

FIGS. 28 and 29 illustrate an alternative embodiment of the capsule 38 wherein the capsule is not connected to the delivery catheter 36. Instead, the capsule 38 is a standalone flexible, woven capsule with pull wires 132 connected to its distal end 130, as shown in FIG. 28. The capsule includes a tapered configuration 136 at its proximal end that is sized to fit in the distal end of the tubular wall structure of the delivery catheter 36. The prosthetic valve can be crimped into the standalone capsule 38.

FIG. 28 shows the capsule 38 with the prosthetic valve mounted on the delivery catheter 36, including the nosecone 134 applied to the distal end of the assembly. FIG. 29 shows the pull wires 132 being pulled back to fold the capsule 38 into a crumpled, sock-like configuration to deploy the prosthetic valve. Advantageously, the standalone capsule 38 does not need a proximal support, reducing the working length of the assembly within the left ventricle.

The sock-like capsules 38 can have varying diameters to adapt them to the shape of different types of implants.

Other advantages of the sock-like capsule 38 configurations include low deployment forces. Only pulling one end of the tube generates less friction than pulling the whole woven tube over the prosthetic valve. In contrast, conventional pulling of the whole capsule generates friction between the capsule surface and the entire prosthetic valve. For sock-like capsules, the pulling point at one end only peels the capsule off the valve frame with folding, rather than relative sliding. Another advantage includes wire pulling through the nosecone 134, reducing or eliminating the need for the nosecone to move forward toward the apex of the left ventricle. This can reduce the risk of chordae entanglement.

The sock-like capsule 38 can also simplify the valve crimping process and reduce the delivery system profile. Only wires without a pulling tube need to go through the delivery catheter 36. Also, the capsule 38 can be retrieved back into the delivery catheter by folding, thus not requiring the delivery system position and orientation to change within the heart. Generally, also, capsules comprised of flexible, foldable materials can be easily reduced in diameter and can be pulled through sharp bends can be much larger in diameter.

In view of the many possible embodiments to which the principles of the disclosed invention can be applied, it should be recognized that the illustrated embodiments are only preferred examples of the disclosure and should not be taken as limiting the scope of the invention. Rather, the scope of the invention is defined by the following claims.

## Claims

1. A system for treating a native heart valve, comprising:
a catheter (36) configured to extend through the vasculature and having a proximal end and a distal end, the distal end of the catheter (36) for positioning in proximity to the native heart valve;
an expandable prosthetic heart valve (78) coupled to the distal end of the catheter (36), the expandable prosthetic valve including a cylindrical frame (80) and at least one paddle (82);
a tubular capsule (38) comprising a proximal end and a distal end, the tubular capsule (38) comprising a flexible wall material having an inner layer (84) extending around the cylindrical frame (80) and an outer layer (86) extending around the paddle (82); and
a pulling element attached to the proximal end of the tubular capsule (38) and extending proximally along the catheter (36);
wherein pulling proximally on the tubular capsule (38) using the pulling element first releases the paddle (82) and then releases the cylindrical frame (80).

2. The system of claim 1, wherein the inner layer (84) has a length greater than the outer layer (86).

3. The system of any one of claims 1 or 2, wherein the inner layer (84) has a crumple zone (89) configured to be taken up without movement of a distal end of the inner layer (84) while the outer layer (86) releases the paddle (82); and
wherein the distal end of the inner layer (84) moves once the crumple zone (89) is taken up to release the cylindrical frame (80).

4. The system of claim 3, wherein, in a first stage of withdrawal of the capsule (38) into the distal end of the catheter (36), the outer layer (86) withdraws in proportion to the length of pull on the proximal end of the capsule (38) while a distal free edge of the inner layer (84) remains static as the crumple zone (89) of the proximal portion straightens out.

5. The system of claim 4, wherein movement of the outer layer (86) releases the paddle (82).

6. The system of claim 5, wherein, in a subsequent stage of withdrawal of the capsule (38) into the distal end of the catheter (36), proximal pulling of the capsule (38) pulls a free end of the inner layer (84) from the outside of the stent body so that it can expand into place, such as within the native mitral valve annulus.

7. The system of claim 1, wherein the outer layer (86) restrains the paddles (82) in position and the inner layer (84) restrains the frame in a crimped position.

8. The system of claim 7, wherein, in a delivery configuration, the inner layer (84) includes a distal portion that is straight in cross section and cylindrical, extending directly under the paddles (82).

9. The system of claim 8, wherein, at the proximal end of the capsule (38), the inner and outer layers (86) are bonded, formed or otherwise coupled together to become a single layer which can be pulled to remove the capsule (38).

10. The system of claim 1, wherein the inner layer (84) has a free end and the outer layer (86) has a free end.

11. The system of claim 1, wherein the tubular capsule (38) extends distally to a distal bend (92) and extends proximally from the distal bend (92) to define the outer layer (86) to a proximal bend (96) then extends distally from the proximal bend (96) under the paddle (82) and over the cylindrical frame (80) to define the inner layer (84).

12. The system of claim 11, wherein pulling proximally on the capsule (38) undoes the distal bend (92), and then proximal bend (96), to remove the outer layer (86) and release the paddles (82); and
wherein pulling retracts the remaining inner layer (84) from the frame so that it can expand into place within a mitral valve annulus.

13. The system of any one of claims 3 or 11, wherein the capsule (38) can be retrieved back into the catheter (36) without changing the delivery system position and orientation within the heart.

14. The system of any one of the preceding claims, wherein the at least one paddle (82) is configured to engage or anchor into the chordae tendineae (18).

15. The system of any one of the preceding claims, wherein the prosthetic heart valve (78) is a self-expanding prosthetic heart valve.

## Patentansprüche

1. System zur Behandlung einer nativen Herzklappe, umfassend:
einen Katheter (36), der dazu konfiguriert ist, sich durch das Gefäßsystem zu erstrecken, und der ein proximales Ende und ein distales Ende aufweist, wobei das distale Ende des Katheters (36) zur Positionierung in der Nähe der nativen Herzklappe dient;
eine expandierbare Herzklappenprothese (78), die mit dem distalen Ende des Katheters (36) gekoppelt ist, wobei die expandierbare Klappenprothese einen zylindrischen Rahmen (80) und wenigstens ein Paddel (82) umfasst;
eine rohrförmige Kapsel (38), die ein proximales Ende und ein distales Ende umfasst, wobei die rohrförmige Kapsel (38) ein flexibles Wandmaterial umfasst, das eine innere Schicht (84), die sich um den zylindrischen Rahmen (80) erstreckt, und eine äußere Schicht (86), die sich um das Paddel (82) erstreckt, aufweist; und
ein Zugelement, das an dem proximalen Ende der rohrförmigen Kapsel (38) befestigt ist und sich proximal entlang des Katheters (36) erstreckt;
wobei ein proximales Ziehen an der rohrförmigen Kapsel (38) mittels des Zugelements zunächst das Paddel (82) freisetzt und anschließend den zylindrischen Rahmen (80) freisetzt.

2. System nach Anspruch 1, wobei die innere Schicht (84) eine größere Länge aufweist als die äußere Schicht (86).

3. System nach einem der Ansprüche 1 oder 2, wobei die innere Schicht (84) eine Knautschzone (89) aufweist, die dazu konfiguriert ist, in Anspruch genommen zu werden, ohne dass sich ein distales Ende der inneren Schicht (84) bewegt, während die äußere Schicht (86) das Paddel (82) freigibt; und
wobei sich das distale Ende der inneren Schicht (84) bewegt, sobald die Knautschzone (89) in Anspruch genommen worden ist, um den zylindrischen Rahmen (80) freizugeben.

4. System nach Anspruch 3, wobei in einer ersten Stufe des Rückzugs der Kapsel (38) in das distale Ende des Katheters (36) die äußere Schicht (86) proportional zur Zugstrecke am proximalen Ende der Kapsel (38) zurückgezogen wird, während ein distaler freier Rand der inneren Schicht (84) statisch bleibt, während sich die Knautschzone (89) des proximalen Abschnitts streckt.

5. System nach Anspruch 4, wobei eine Bewegung der äußeren Schicht (86) das Paddel (82) freigibt.

6. System nach Anspruch 5, wobei in einer nachfolgenden Stufe des Rückzugs der Kapsel (38) in das distale Ende des Katheters (36) ein proximales Ziehen der Kapsel (38) ein freies Ende der inneren Schicht (84) von der Außenseite des Stentkörpers abzieht, so dass dieser sich an Ort und Stelle entfalten kann, beispielsweise innerhalb des nativen Mitralklappenanulus.

7. System nach Anspruch 1, wobei die äußere Schicht (86) die Paddel (82) in ihrer Stellung hält und die innere Schicht (84) den Rahmen in einer gecrimpten Stellung hält.

8. System nach Anspruch 7, wobei in einer Zuführkonfiguration die innere Schicht (84) einen distalen Abschnitt aufweist, der im Querschnitt gerade und zylindrisch ist und sich unmittelbar unterhalb der Paddel (82) erstreckt.

9. System nach Anspruch 8, wobei am proximalen Ende der Kapsel (38) die innere und die äußere Schicht (86) miteinander verbunden, geformt oder anderweitig gekoppelt sind, um eine einzelne Schicht zu bilden, die gezogen werden kann, um die Kapsel (38) zu entfernen.

10. System nach Anspruch 1, wobei die innere Schicht (84) ein freies Ende aufweist und die äußere Schicht (86) ein freies Ende aufweist.

11. System nach Anspruch 1, wobei die rohrförmige Kapsel (38) sich distal von einer distalen Biegung (92) erstreckt und sich proximal von der distalen Biegung (92) erstreckt, um die äußere Schicht (86) bis zu einer proximalen Biegung (96) zu definieren, und sich dann distal von der proximalen Biegung (96) unterhalb des Paddels (82) und über den zylindrischen Rahmen (80) erstreckt, um die innere Schicht (84) zu definieren.

12. System nach Anspruch 11, wobei ein proximales Ziehen an der Kapsel (38) die distale Biegung (92) und anschließend die proximale Biegung (96) aufhebt, um die äußere Schicht (86) zu entfernen und die Paddel (82) freizugeben; und
wobei das Ziehen die verbleibende innere Schicht (84) vom Rahmen zurückzieht, so dass dieser sich an Ort und Stelle innerhalb eines Mitralklappenanulus entfalten kann.

13. System nach einem der Ansprüche 3 oder 11, wobei die Kapsel (38) in den Katheter (36) zurückgezogen werden kann, ohne die Position und Ausrichtung des Zuführsystems innerhalb des Herzens zu verändern.

14. System nach einem der vorhergehenden Ansprüche, wobei das wenigstens eine Paddel (82) dazu konfiguriert ist, in die Chordae Tendineae (18) einzugreifen oder in diesen zu ankern.

15. System nach einem der vorhergehenden Ansprüche, wobei die Herzklappenprothese (78) eine selbstexpandierende Herzklappenprothese ist.

## Revendications

1. Système de traitement d'une valvule cardiaque native, comprenant :
un cathéter (36) conçu pour s'étendre à travers le système vasculaire et présentant une extrémité proximale et une extrémité distale, l'extrémité distale du cathéter (36) étant destinée à être positionnée à proximité de la valvule cardiaque native ;
une valvule cardiaque prothétique (78) déployable accouplée à l'extrémité distale du cathéter (36), la valvule prothétique déployable comportant un cadre cylindrique (80) et au moins une palette (82) ;
une capsule tubulaire (38) comprenant une extrémité proximale et une extrémité distale, la capsule tubulaire (38) comprenant un matériau de paroi souple présentant une couche interne (84) s'étendant autour du cadre cylindrique (80) et une couche externe (86) s'étendant autour de la palette (82) ; et
un élément de traction fixé à l'extrémité proximale de la capsule tubulaire (38) et s'étendant de manière proximale le long du cathéter (36) ;
dans lequel une traction de manière proximale sur la capsule tubulaire (38) à l'aide de l'élément de traction libère d'abord la palette (82) puis libère le cadre cylindrique (80).

2. Système selon la revendication 1, dans lequel la couche interne (84) présente une longueur supérieure à celle de la couche externe (86).

3. Système selon l'une quelconque des revendications 1 ou 2, dans lequel la couche interne (84) présente une zone déformable (89) conçue pour être absorbée sans déplacement d'une extrémité distale de la couche interne (84) tandis que la couche externe (86) libère la palette (82) ; et
dans lequel l'extrémité distale de la couche interne (84) se déplace une fois que la zone déformable (89) est absorbée afin de libérer le cadre cylindrique (80).

4. Système selon la revendication 3, dans lequel, dans une première étape de retrait de la capsule (38) dans l'extrémité distale du cathéter (36), la couche externe (86) se retire proportionnellement à la longueur de traction sur l'extrémité proximale de la capsule (38) tandis qu'un bord libre distal de la couche interne (84) reste statique à mesure que la zone déformable (89) de la partie proximale s'étire.

5. Système selon la revendication 4, dans lequel le déplacement de la couche externe (86) libère la palette (82).

6. Système selon la revendication 5, dans lequel, dans une étape subséquente de retrait de la capsule (38) dans l'extrémité distale du cathéter (36), une traction proximale de la capsule (38) tire une extrémité libre de la couche interne (84) depuis l'extérieur du corps d'endoprothèse de telle sorte qu'il peut se déployer en place, par exemple à l'intérieur de l'anneau de valvule mitrale native.

7. Système selon la revendication 1, dans lequel la couche externe (86) retient les palettes (82) en position et la couche interne (84) retient le cadre dans une position comprimée.

8. Système selon la revendication 7, dans lequel, dans une configuration de pose, la couche interne (84) comporte une partie distale qui est droite en section transversale et cylindrique, s'étendant directement sous les palettes (82).

9. Système selon la revendication 8, dans lequel, au niveau de l'extrémité proximale de la capsule (38), les couches interne et externe (86) sont liées, formées ou autrement accouplées ensemble afin de devenir une couche unique qui peut être tirée en vue d'enlever la capsule (38).

10. Système selon la revendication 1, dans lequel la couche interne (84) présente une extrémité libre et la couche externe (86) présente une extrémité libre.

11. Système selon la revendication 1, dans lequel la capsule tubulaire (38) s'étend de manière distale jusqu'à un coude distal (92) et s'étend de manière proximale à partir du coude distal (92) afin de définir la couche externe (86) vers un coude proximal (96) puis s'étend de manière distale à partir du coude proximal (96) sous la palette (82) et sur le cadre cylindrique (80) afin de définir la couche interne (84).

12. Système selon la revendication 11, dans lequel la traction de manière proximale sur la capsule (38) défait le coude distal (92), puis le coude proximal (96), afin d'enlever la couche externe (86) et de libérer les palettes (82) ; et
dans lequel une traction rétracte la couche interne (84) restante à partir du cadre de telle sorte qu'il peut se déployer en place à l'intérieur d'un anneau de valvule mitrale.

13. Système selon l'une quelconque des revendications 3 ou 11, dans lequel la capsule (38) peut être récupérée dans le cathéter (36) sans modifier la position et l'orientation du système de pose à l'intérieur du cœur.

14. Système selon l'une quelconque des revendications précédentes, dans lequel ladite au moins une palette (82) est conçue pour venir en prise avec les cordages tendineux (18) ou s'ancrer dans ceux-ci.

15. Système selon l'une quelconque des revendications précédentes, dans lequel la valvule cardiaque prothétique (78) est une valvule cardiaque prothétique autodéployable.
